# EUROPÄISCHE PATENTSCHRIFT

(11) **EP 1 343 786 B1**
(45) Veröffentlichungstag und Bekanntmachung des Hinweises auf die Patenterteilung: **29.06.2005**
(21) Anmeldenummer: 01997489.8
(22) Anmeldetag: 09.11.2001
(51) Int. Cl.: C07D 471/04, A61K 31/415, A61P 9/00

(54) **NEUE PYRIDIN-SUBSTITUIERTE PYRAZOLOPYRIDINDERIVATE**
NOVEL PYRIDINE-SUBSTITUTED PYRAZOLOPYRIDINE DERIVATIVES
NOUVEAUX DERIVES DE PYRAZOLOPYRIDINE A SUBSTITUTION PYRIDINE

(30) Priorität: 22.11.2000 DE 10057753; 02.07.2001 DE 10131987
(43) Veröffentlichungstag der Anmeldung: 17.09.2003
(73) Patentinhaber: Bayer HealthCare AG, 51368 Leverkusen (DE)
(72) Erfinder: STASCH, Johannes-Peter, 42651 Solingen (DE); FEURER, Achim, 69259 Wilhelmsfeld (DE); WEIGAND, Stefan, 42115 Wuppertal (DE); STAHL, Elke, 51467 Bergisch Gladbach (DE); FLUBACHER, Dietmar, 79110 Freiburg (DE); ALONSO-ALIJA, Cristina, 42781 Haan (DE); WUNDER, Frank, 42117 Wuppertal (DE); LANG, Dieter, 42553 Velbert (DE); DEMBOWSKY, Klaus, Boston, MA 02116 (US); STRAUB, Alexander, 42117 Wuppertal (DE); PERZBORN, Elisabeth, 42327 Wuppertal (DE)
(86) Internationale Anmeldenummer: PCT/EP2001/012969
(87) Internationale Veröffentlichungsnummer: WO 2002/042301

(56) Entgegenhaltungen:
- DE-A- 10 021 069
- DE-A- 19 834 044
- DE-A- 19 834 045
- DE-A- 19 834 047
- DE-A- 19 920 352
- STRAUB A ET AL: "NO-Independent stimulators of soluble guanylate cyclase" BIOORGANIC & MEDICINAL CHEMISTRY LETTERS, OXFORD, GB, Bd. 11, Nr. 6, 26. März 2001 (2001-03-26), Seiten 781-784, XP004230931 ISSN: 0960-894X

## Beschreibung

Die vorliegende Erfindung betrifft neue chemische Verbindungen, welche die lösliche Guanylatcyclase stimulieren, ihre Herstellung und ihre Verwendung als Arzneimittel, insbesondere als Arzneimittel zur Behandlung von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NOunabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Br.J.Pharmacol. 120 (1997), 681), Fettsäuren (Goldberg et al, J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587) sowie verschiedene substituierte Pyrazolderivate (WO 98/16223).

Weiterhin sind in der WO 98/16507, WO 98/23619, WO 00/06567, WO 00/06568, WO 00/06569 und WO 00/21954 Pyrazolopyridinderivate als Stimulatoren der löslichen Guanylatcyclase beschrieben. In diesen Patentanmeldungen sind auch Pyrazolopyridine beschrieben, welche einen Pyrimidinrest in 3-Position aufweisen. Derartige Verbindungen weisen eine sehr hohe in vitro Aktivität bezüglich der Stimulation der löslichen Guanylatcyclase auf Allerdings zeigte es sich, dass diese Verbindungen hinsichtlich ihrer in vivo-Eigenschaften wie beispielsweise ihrem Verhalten in der Leber, ihrem pharmakokinetischen Verhalten, ihrer Dvsis-Wirkungsbeziehung oder ihrem Metabolisierungsweg einige Nachteile aufweisen.

Es war daher die Aufgabe der vorliegenden Erfindung, weitere Pyrazolopyridinderivate bereitzustellen, welche als Stimulatoren der löslichen Guanylatcyclase wirken, aber nicht die vorstehend aufgeführten Nachteile der Verbindungen aus dem Stand der Technik aufweisen.

Diese Aufgabe wird gemäß der vorliegenden Erfindung durch die Verbindungen gemäß Anspruch 1 gelöst. Diese neuen Pyrazolopyridinderivate zeichnen sich durch einen Pyrimidinrest in 3-Position aus, der ein bestimmtes Substitutionsmuster aufweist, nämlich einen Pyridinrest in 5-Position des Pyrimidinrings sowie eine Aminogruppe in 4-Position des Pyrimidinrings.

Im einzelnen betrifft die vorliegende Erfindung die Verbindungen der Formel (I) 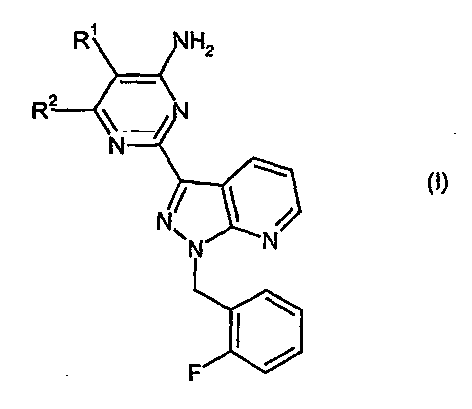 worin
- R¹: für 4-Pyridinyl oder 3-Pyridinyl steht;
- R²: für H, NH₂ oder Halogen steht;
sowie Salze, Isomere und Hydrate davon.

Gemäß einer alternativen Ausführungsform betrifft die vorliegende Erfindung Verbindungen der Formel (I), bei denen
- R¹: für 4-Pyridinyl oder 3-Pyridinyl steht;
- R²: für H, NH₂ oder Cl steht;
sowie Salze, und Hydrate davon.

Gemäß einer weiteren alternativen Ausführungsform betrifft die vorliegende Erfindung Verbindungen der Formel (I), bei denen
- R¹: für 4-Pyridinyl oder 3-Pyridinyl steht;
- R²: für H steht;
sowie Salze, und Hydrate davon.

Die erfindungsgemäßen Verbindungen der Formel (I) können auch in Form ihrer Salze vorliegen. Im allgemeinen seien hier Salze mit organischen oder anorganischen Basen oder Säuren genannt

Im Rahmen der vorliegenden Erfindung werden physiologisch unbedenkliche Salze bevorzugt. Physiologisch unbedenkliche Salze der erfindungsgemäßen Verbindung können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindung sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in tautomeren Formen vorliegen. Dies ist dem Fachmann bekannt, und derartige Formen sind ebenfalls vom Umfang der Erfindung umfasst.

Weiterhin können die erfindungsgemäßen Verbindungen in Form ihrer möglichen Hydrate vorkommen.

Halogen steht im Rahmen der Erfindung für Fluor, Chlor, Brom und Iod.

Die erfindungsgemäßen Verbindungen der Formel (I) können hergestellt werden durch die Umsetzung der Verbindung der Formel (II) 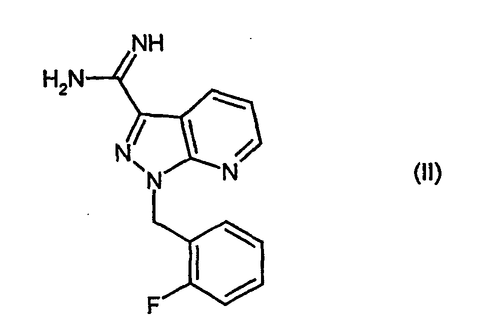
A) mit einer Verbindung der Formel (III) 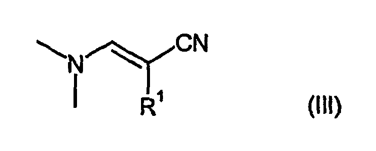 wobei
   - R¹: wie vorstehend definiert ist;
   gegebenenfalls in einem organischen Lösungsmittel unter Erhitzen zur Verbindung der Formel (I);
   oder
B) mit einer Verbindung der Formel (IV) 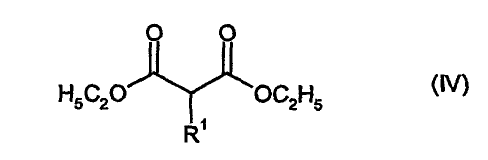
wobei
- R¹: wie vorstehend definiert ist;
in einem organischen Lösungsmittel unter Erhitzen zu Verbindungen der Formel (V) 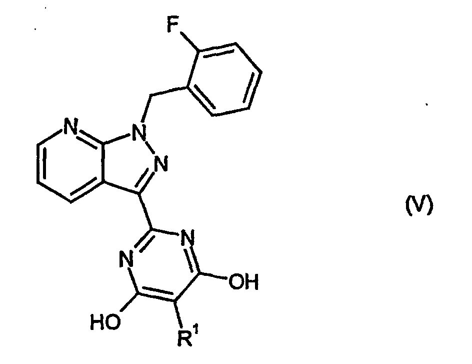 wobei
- R¹: wie vorstehend definiert ist;
anschließend mit einem Halogenierungsmittel zu Verbindungen der Formel (VI) 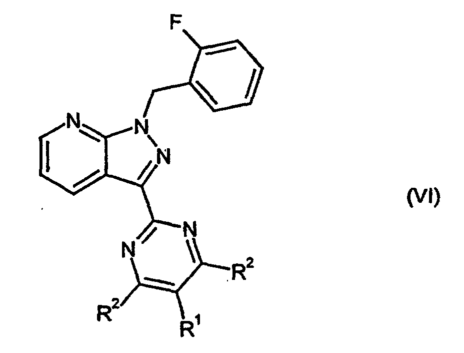 wobei
- R¹: wie vorstehend definiert ist;
- R²: für Halogen steht;
sowie abschließend mit wäßriger Ammoniaklösung unter Erhitzen und erhöhtem Druck.

Die Verbindung der Formel (II) lässt sich gemäß folgendem Reaktionsschema herstellen:

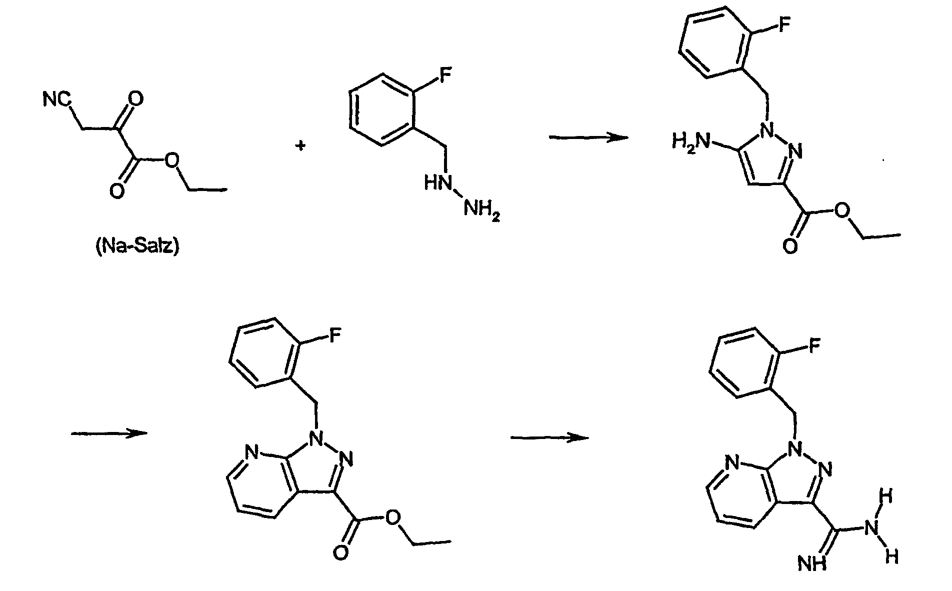

Die Verbindung der Formel (II) ist in einer mehrstufigen Synthese aus dem literaturbekannten Natriumsalz des Cyanobrenztraubensäureethylesters (Borsche und Manteuffel, Liebigs. Ann. Chem. 1934, 512, 97) erhältlich. Durch dessen Umsetzung mit 2-Fluorbenzylhydrazin unter Erhitzen und Schutzgasatmosphäre in einem inerten Lösungsmittel wie Dioxan erhält man den 5-Amino-1-(2-fluorbenzyl)-pyrazol-3-carbonsäureethylester, der durch Umsetzung mit Dimethylaminoacrolein im sauren Medium unter Schutzgasatmosphäre und Erhitzen zum entsprechenden Pyridinderivat cyclisiert. Dieses Pyridinderivat 1-(2-Fluorbenzyl)-1H-pyrazolo[3,4-b]pyridin-3-carbonsäureethylester wird durch eine mehrstufige Sequenz, bestehend aus Überführung des Esters mit Ammoniak in das entsprechende Amid, Dehydratisierung mit einem wasserentziehenden Mittel wie Trifluoressigsäureanhydrid zum entsprechenden Nitrilderivat, Umsetzung des Nitrilderivats mit Natriumethylat und abschließende Reaktion mit Ammoniumchlorid in die Verbindung der Formel (II) überführt.

Die Verbindungen der Formel (III) können aus den (z.B. bei Aldrich) käuflich erhältlichen Verbindungen t-Butoxybis(dimethylamino)methan und 4-Pyridylacetonitril beziehungsweise 3-Pyridylacetonitril durch Umsetzung dieser Reaktanden vorzugsweise in äquimolaren Mengen vorzugsweise bei Normaldruck und Rühren der Reaktionslösung für mehrere Stunden, beispielsweise 2 Stunden, bei erhöhter Temperatur, beispielsweise 60-130°C, vorzugsweise 80-120°C, insbesondere 100°C hergestellt werden.

Die Umsetzung der Verbindungen der Formeln (II) und (III) zu den Verbindungen der Formel (I) kann durch Einsatz der Reaktanden in äquimolaren Mengen beziehungsweise unter Verwendung der Verbindung der Formel (III) im leichten Überschuss in einem organischen Lösungsmittel, beispielsweise einem Kohlenwasserstoff, vorzugsweise einem aromatischen Kohlenwasserstoff und insbesondere Xylol, vorzugsweise in Gegenwart von 0,1-1 Äquivalenten, vorzugsweise 0,3 Äquivalenten einer Lewis-Säure wie beispielsweise BF₃^{.}Et₂O oder Trimethylsilyltrifluorsulfonat (TMSOTf), vorzugsweise bei Normaldruck und Rühren der Reaktionslösung für mehrere Stunden, beispielsweise 12 Stunden, bei erhöhter Temperatur, beispielsweise 80-160°C, vorzugsweise 100-150°C, insbesondere 140°C, durchgeführt werden.

Die Verbindungen der Formel (IV) sind kommerziell erhältlich (z.B. bei Mercachem) oder können auf dem Fachmann bekannte Weise dargestellt werden.

Die Umsetzung der Verbindungen der Formeln (II) und (IV) zu den Verbindungen der Formel (V) kann durch Einsatz der Reaktanden in äquimolaren Mengen beziehungsweise unter Verwendung der Verbindung der Formel (IV) im leichten Überschuss in einem organischen Lösungsmittel, beispielsweise einem Kohlenwasserstoff, vorzugsweise einem aromatischen Kohlenwasserstoff und insbesondere Toluol, vorzugsweise bei Normaldruck und Rühren der Reaktionslösung für mehrere Stunden, beispielsweise 12 Stunden, bei erhöhter Temperatur, beispielsweise 80-160°C, vorzugsweise 100-150°C, insbesondere 140°C, durchgeführt werden.

Die Umsetzung der Verbindungen der Formel (V) zu Verbindungen der Formel (VI) kann durch Reaktion der Verbindungen der Formel (V) mit einem Halogenierungsmittel, gegebenenenfalls in einem für derartige Reaktionen herkömmlich verwendeten organischen Lösungsmittel wie beispielsweise Dimethylformamid (DMF), vorzugsweise bei Normaldruck und Rühren der Reaktionslösung für mehrere Stunden, beispielsweise 3 Stunden, bei erhöhter Temperatur, beispielsweise 80-160°C, vorzugsweise 100-120°C, durchgeführt werden. Erfindungsgemäß bevorzugt kann als Halogenierungsmittel POCl₃ eingesetzt werden.

Die Umsetzung der Verbindungen der Formel (VI) zu den erfindungsgemäßen Verbindungen der Formel (I) kann durch Reaktion der Verbindungen der Formel (VI) mit wäßriger Ammoniaklösung vorzugsweise bei erhöhtem Druck, beispielsweise durch Ablauf der Reaktion in einem Autoklaven so dass die Reaktion unter dem Eigendruck der Reaktionsmischung verläuft, und Rühren der Reaktionslösung für mehrere Stunden, beispielsweise 12 Stunden, bei erhöhter Temperatur beispielsweise 80-160°C, vorzugsweise 100-150°C, insbesondere 140°C, durchgeführt werden.

Die erfindungsgemäßen Verbindungen der Formel (I) zeigt ein nicht vorhersehbares, wertvolles pharmakologisches Wirkspektrum.

Die erfindungsgemäßen Verbindungen der Formel (I) führen zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärkt die erfindungsgemäße Verbindung der Formel (I) die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektile Dysfunktion, weibliche sexuelle Dysfunktion, Osteoporose, Gastroparese und Inkontinenz eingesetzt werden.

Die in der vorliegenden Erfindung beschriebenen Verbindungen der Formel (I) stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung, oder Gedächtnisleistung nach kognitiven Störungen, wie sie insbesondere bei Situationen/Krankheiten/Syndromen auftreten wie "Mild cognitive impairment", Altersassoziierte Lem- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatisches Schädel Hirn Trauma, allgemeine Konzentrationsstörungen, Konzentrationsstörungen in Kindern mit Lern-und Gedächtnisproblemen, Alzheimersche Krankheit, Vaskuläre Demenz, Demenz mit Lewy-Körperchen, Demenz mit Degeneration der Frontallappen einschliesslich des Pick's Syndroms, Parkinsonsche Krankheit, Progressive nuclear palsy, Demenz mit corticobasaler Degeneration, Amyolateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuss- und Suchtmittelaufnahme.

Weiterhin eignet sich die Wirkstoffe auch zur Regulation der cerebralen Durchblutung und stellt somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen der Formel (I) zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Darüber hinaus umfasst die Erfindung die Kombination der erfindungsgemäßen Verbindungen der Formel (I) mit organischen Nitraten und NO-Donatoren.

Organische Nitrate und NO-Donatoren im Rahmen der Erfindung sind im allgemeinen Substanzen, die über die Freisetzung von NO bzw. NO-Species ihre therapeutische Wirkung entfalten. Bevorzugt sind Natriumnitroprussid, Nitroglycerin, Isosorbiddinitrat, Isosorbidmononitrat, Molsidomin und SIN-1.

Außerdem umfasst die Erfindung die Kombination mit Verbindungen, die den Abbau von cyclischem Guanosinmonophosphat (cGMP) inhibieren. Dies sind insbesondere Inhibitoren der Phosphodiesterasen 1, 2 und 5; Nomenklatur nach Beavo und Reifsnyder (1990) TiPS 11 S. 150 bis 155. Durch diese Inhibitoren wird die Wirkung der erfindungsgemäßen Verbindungen potenziert und der gewünschte pharmakologische Effekt gesteigert.

### Biologische Untersuchungen

### Gefäßrelaxierende Wirkung in vitro

Kaninchen werden durch Nackenschlag betäubt und entblutet. Die Aorta wird entnommen, von anhaftendem Gewebe befreit, in 1,5 mm breite Ringe geteilt und einzeln unter einer Vorspannung in 5 ml-Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x 7 H₂O; 1,4; KH₂PO₄: 1,2; NaHCO₃:25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung untersucht und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50 % zu reduzieren (IC₅₀). Das Standardapplikationsvolumen beträgt 5 µl, der DMSO-Anteil in der Badlösung entspricht 0,1 %. Das Ergebnis ist nachstehend in Tabelle 1 aufgeführt:

**Tabelle 1:**

| **Geläßrelaxierende Wirkung in vitro** | |
|---|---|
| Beispiel Nr. | IC₅₀ [µM] |
| 1 | 0,66 |
| 4 | 1,21 |

### Bestimmung der Leberclearance in vitro

Ratten werden anästhesiert, heparinisiert, und die Leber in situ über die Pfortader perfundiert. Ex vivo werden dann aus der Leber mittels Collagenase-Lösung die primären Ratten-Hepatozyten gewonnen. Es wurden 2^{.}10⁶ Hepatozyten pro ml mit jeweils der gleichen Konzentration der zu untersuchenden Verbindung bei 37°C inkubiert. Die Abnahme des zu untersuchenden Substrates über die Zeit wurde bioanalytisch (HPLC/UV, HPLC/Fluoreszenz oder LC/MSMS) an jeweils 5 Zeitpunkten im Zeitraum von 0-15 min nach Inkubationsstart bestimmt. Daraus wurde über Zellzahl und Lebergewicht die Clearance errechnet.

### Bestimmung der Plasmaclearance in vivo

Die zu untersuchende Substanz wird Ratten über die Schwanzvene intravenös als Lösung appliziert. Zu festgelegten Zeitpunkten wird den Ratten Blut entnommen, dieses wird heparinisiert und durch herkömmliche Maßnahmen Plasma daraus gewonnen. Die Substanz wird im Plasma bioanalytisch quantifiziert. Aus den so ermittelten Plasmakonzentrations-Zeit-Verläufen werden über herkömmliche hierfür verwendete nicht-kompartimentelle Methoden die pharmakokinetischen Parameter errechnet.

Zur vorliegenden Erfindung gehören pharmazeutische Zubereitungen, die neben nichttoxischen, inerten pharmazeutisch geeigneten Trägerstoffen die erfindungsgemäße Verbindung der Formel (I) enthält sowie Verfahren zur Herstellung dieser Zubereitungen.

Der Wirkstoff kann gegebenenfalls in einem oder mehreren der oben angegebenen Trägerstoffe auch in mikroverkapselter Form vorliegen.

Die therapeutisch wirksame Verbindung der Formel (I) soll in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer der erfindungsgemäßen Verbindung der Formel (I) auch weitere pharmazeutische Wirkstoffe enthalten.

Im allgemeinen hat es sich sowohl in der Human- als auch in der Veterinärmedizin als vorteilhaft erwiesen, den erfindungsgemäßen Wirkstoff in Gesamtmengen von etwa 0,01 bis etwa 700, vorzugsweise 0,01 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den erfindungsgemäßen Wirkstoff vorzugsweise in Mengen von etwa 0,1 bis etwa 80, insbesondere 0,1 bis 30 mg/kg Körpergewicht.

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Beispiele

| Abkürzungen: | |
|---|---|
| RT | Raumtemperatur |
| EE | Essigsäureethylester |
| MCPBA | m-Chlorperoxybenzoesäure |
| BABA | n-Butylacetat/n-Butanol/Eisessig/Phosphatpuffer pH 6 (50:9:25.15; org. Phase) |
| DMF | N,N-Dimethylformamid |

| Laufmittel für die Dünnschichtchromatographie: | |
|---|---|
| T1 E1 | Toluol - Essigsäureethylester (1:1) |
| T1 EtOH1 | Toluol - Methanol (1:1) |
| C1 E1 | Cyclohexan - Essigsäureethylester (1:1) |
| C1 E2 | Cyclohexan - Essigsäureethylester (1:2) |

### Methoden zur Ermittlung der HPLC-Retentionszeiten bzw. präparative Trennmethoden:

*Methode A = (LC-MS):*

| | |
|---|---|
| Eluent | A = Acetonitril + 0.1% Ameisensäure, |
| | B = Wasser + 0.1% Ameisensäure |
| Fluß | 25 ml/min |
| Temperatur | 40°C |
| Packungsmaterial | Symmetry C 18, 50x2.1 mm, 3.5 µm. |

| Zeit(min) | A | B |
|---|---|---|
| 0 | 10 | 90 |
| 4.0 | 90 | 10 |
| 6.0 | 90 | 10 |
| 6.1 | 10 | 90 |
| 7.5 | 10 | 90 |

*Methode B (präparative HPLC):*

| | |
|---|---|
| Eluent | A = Milli-Q-Wasser + 0.6g konzentrierte Salzsäure auf 11 H₂O |
| | B = Acetonitril |
| Fluß | 50 ml/min |
| Temperatur | Raumtemperatur |
| Packungsmaterial | YMC-Gel ODS-AQS 11µm 250 x 30 mm |

| Zeit(min) | A | B |
|---|---|---|
| 0 | 90 | 10 |
| 3 | 90 | 10 |
| 27 | 2 | 98 |
| 34 | 2 | 98 |
| 34.01 | 90 | 10 |
| 38 | 90 | 10 |

### Ausgangsverbindungen:

### I. Synthese von 4-[(Dimethylamino)methylen]-pyridinacetonitril (E/Z-Gemisch)

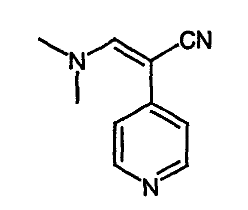

4-Pyridylacetonitril 7.52 g (63.7 mmol) und tert-Butoxybis(dimethylamino)methan 11.09 g (63.7 mmol) wurden bei 100°C für 2 h gerührt. Dabei wurde frei werdendes Dimethylamin und t-Butanol mittels einer Vakuumpumpe durch leichten Unterdruckstrom zur Atmosphäre abgeführt. Flash-Chromatographie (CH₂Cl₂/Ethylacetat 50:1 → 20:1) lieferte die Titelverbindung.
- Ausbeute:: 10.2 g (93 %)
- R_{f}-Wert:: 0.29 (CH₂Cl₂/EE 20/1)
- ¹H-NMR:: (300 MHz, D₆-DMSO), δ = 3.25 (s, 6 H, 2 x CH₃), 7.25 (d, 2 H, Ar-H), 7.80 (s, 1 H, Ar-H), 8.33 (d, 2 H, Ar-H).
- MS:: (ESI pos.), *m*/*z* = 174 ([M+H]⁺)

### II. Synthese von 3-[(Dimethylamino)methylen]-pyridinacetonitril (E/Z-Gemisch)

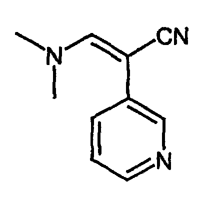

3-Pyridylacetonitril 3.00 g (25.4 mmol) und tert-Butoxybis(dimethylamino)methan 4.23 g (25.4 mmol) wurden bei 100°C für 2 h gerührt. Dabei wurde frei werdendes Dimethylamin und t-Butanol mittels einer Vakuumpumpe durch leichten Unterdruckstrom zur Atmosphäre abgeführt. Nach dem Abkühlen filtrierte man vom ausgefallenen Feststoff, wusch diesen mit wenig H₂O und erhielt so die Titelverbindung.
- Ausbeute:: 4.23 g (96 %)
- R_{f}-Wert:: 0.27 (CH₂Cl₂/MeOH 40/1)
- ¹H-NMR:: (300 MHz, D₆-DMSO), δ = 3.08 (s, 3 H, CH₃), 3.25 (s, 3 H, CH₃), 7.29 (dd, 1 H, Ar-H), 7.57 (s, 1 H, =C-H), 7.66 (dt, 1 H, Ar-H), 8.26 (d, 1 H, Ar-H), 8.54 (d, 1 H, Ar-H).
- LCMS:: Ret.-zeit: 0.33 min (Säule: Symmetry, C-18, 3.5 µm, 50X2.1 mm, Fluss 0.5 ml/min, 40°C, Gradient: Wasser (+0.1 % Ameisensäure):Acetonitril (+0.1 % Ameisensäure) bei 0 min: 90:10, bei 7.5 min 10:90)); MS: (ESI pos.), *m*/*z* = 174 ([M+H]⁺)

### III. Synthese von 1-(2-Fluorbenzyl)1H-pyrazolo[3,4-b]pyridin-3-carboxamidin

### IIIA) 5-Amino-1-(2-fluorbenzyl)-pyrazol-3-carbonsäureethylester

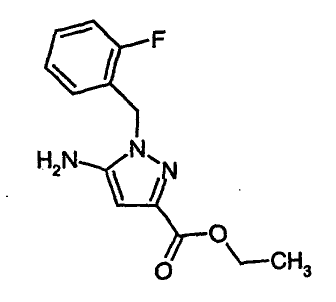

100 g (0.613 mol) Natriumsalz des Cyanobrenztraubensäureethylester (Darstellung analog Borsche und Manteuffel, Liebigs Ann. 1934, *512*, 97) werden unter gutem Rühren unter Argon in 2.5 l Dioxan bei Raumtemperatur mit 111.75 g (75 ml, 0.98 mol) Trifluoressigsäure versetzt und 10 min gerührt, wobei ein großer Teil des Eduktes in Lösung geht. Dann gibt man 85.93 g (0.613 mol) 2-Fluorbenzylhydrazin hinzu und kocht über Nacht. Nach Abkühlen werden die ausgefallenen Kristalle des Natriumtrifluoracetats abgesaugt, mit Dioxan gewaschen und die Lösung roh weiter umgesetzt.

### IIIB) 1-(2-Fluorbenzyl)-1H-pyrazolo[3,4-b]pyridin-3-carbonsäureethylester

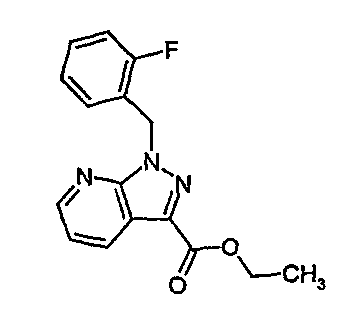

Die aus 3A) erhaltene Lösung wird mit 61.25 ml (60.77 g, 0.613 mol) Dimethylaminoacrolein und 56.28 ml (83.88 g, 0.736 mol) Trifluoressigsäure versetzt und unter Argon 3 Tage lang gekocht. Anschließend wird das Lösungsmittel im Vakuum verdampft, der Rückstand in 2 l Wasser gegeben und dreimal mit je 1 l Essigester extrahiert. Die vereinigten organischen Phasen werden mit Magnesiumsulfat getrocknet und einrotiert. Man chromatographiert auf 2.5 kg Kieselgel und eluiert mit einem Toluol / Toluol-Essigester=4:1 -Gradienten. Ausbeute: 91.6 g (49.9 % d.Th. über zwei Stufen).
Smp. 85°C
R_{f}(SiO₂, T1E1): 0.83

### IIIC) 1-(2-Fluorbenzyl)-1H-pyrazolo[3,4-b]pyridin-3-carboxamid

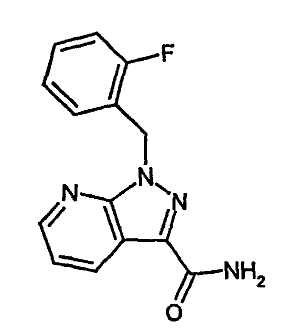

10.18 g (34 mmol) des in Beispiel 3B) erhaltenen Esters werden in 150 ml mit Ammoniak bei 0 - 10°C gesättigtem Methanol vorgelegt. Man rührt zwei Tage bei Raumtemperatur und engt anschließend im Vakuum ein.
R_{f} (SiO₂, T1E1): 0.33

### IIID) 3-Cyano-1-(2-fluorbenzyl)-1H-pyrazolo[3,4-b]pyridin

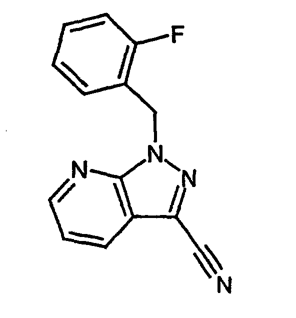

36.1 g (133 mmol) 1-(2-Fluorbenzyl)-1H-pyrazolo[3,4-b]pyridin-3-carboxamid aus Beispiel 3C) werden in 330 ml THF gelöst und mit 27 g (341 mmol) Pyridin versetzt. Anschließend gibt man innerhalb von 10 min 47.76 ml (71.66 g, 341 mmol) Trifluoressigsäureanhydrid hinzu, wobei die Temperatur bis auf 40°C ansteigt. Man rührt über Nacht bei Raumtemperatur. Anschließend wird der Ansatz in 11 Wasser gegeben und dreimal mit je 0.5 1 Essigester extrahiert. Die organische Phase wird mit gesättigter Natriumhydrogencarbonatlösung und mit 1 N HCl gewaschen, mit MgSO4 getrocknet und einrotiert.
Ausbeute: 33.7 g (100 % d.Th.)
Smp: 81°C
R_{f} (SiO₂, T1E1): 0.74

### IIIE) (2-Fluorbenzyl)-1H-pyrazolo[3,4-b]pyridin-3-carboximidsäuremethylester

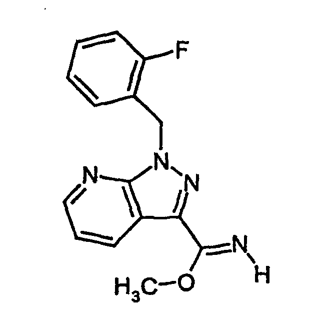

Man löst 30.37 g (562 mmol) Natriummethylat in 1.5 1 Methanol und gibt 36.45 g (144.5 mmol) 3-Cyano-1-(2-fluorbenzyl)-1H-pyrazolo[3,4-b]pyridin (aus Beispiel 3D) hinzu. Man rührt 2 Stunden bei Raumtemperatur und setzt die erhaltene Lösung direkt für die nächste Stufe ein.

### IIIF) 1-(2-Fluorbenzyl)1H-pyrazolo[3,4-b]pyridin-3-carboxamidin

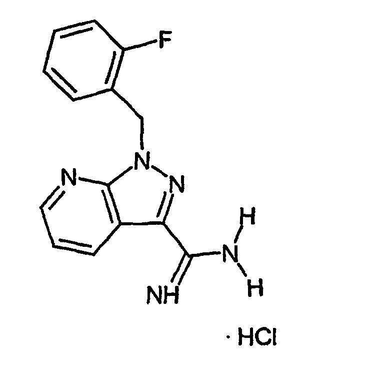

Die aus Beispiel 2E) erhaltene Lösung von (2-Fluorbenzyl)-1H-pyrazolo[3,4-b]pyridin-3-carboximidsäuremethylester in Methanol wird mit 33.76 g (32.19 ml, 562 mmol) Eisessig und 9.28 g (173 mmol) Ammoniumchlorid versetzt und über Nacht unter Rückfluss gerührt. Man verdampft das Lösungsmittel im Vakuum, verreibt den Rückstand gut mit Aceton und saugt den ausgefallenen Feststoff ab.
¹H-NMR (d₆-DMSO, 200 MHz): δ= 5,93 (s, 2H); 7,1-7,5 (m, 4 H); 7,55 (dd, 1H); 8,12 (dd, 1H); 8,30 (dd, 1H); 9,5 (bs, 4H-austauschbar) ppm.
MS (EI): m/z = 270,2 (M-HCl)

### IV. Synthese von 2-[1-(2-Fluorobenzyl)-1H-pyrazolo[3,4-b]pyridin-3-yl]-5-(4-pyridinyl)-4,6-pyrimidindiol

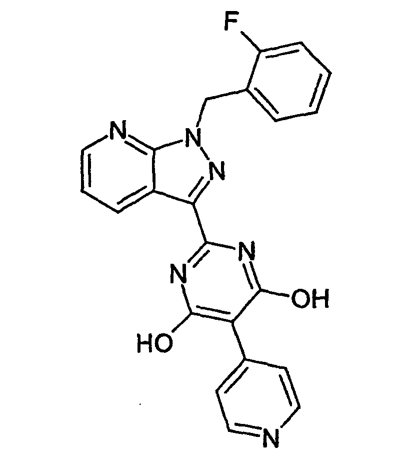

3.27 g (12.1 mmol)1-(2-Fluorobenzyl)-1H-pyrazolo[3,4-b]pyridin-3-carboximidamid aus Bsp. III werden in 40 ml Toluol suspendiert, mit 2.88 g (12.1 mmol) Diethyl 2-(4-pyridinyl)malonat (kommerziell erhältlich bei Mercachem) versetzt und über Nacht bei 140°C gerührt. Zur Aufarbeitung saugt man den ausgefallenen Feststoff ab und trocknet im Hochvakuum.
- Ausbeute:: 2.43 g (43 %)
- LC-MS:: Rₜ = 2.69 min (Methode A).
MS (ESI pos.), *m*/*z* = 415 ([M+H]⁺).

### V. Synthese von 3-[4,6-Dichloro-5-(4-pyridinyl)-2-pyrimidinyl]-1-(2-fluorobenzyl)-1H-pyrazolo-[3,4-b]pyridin

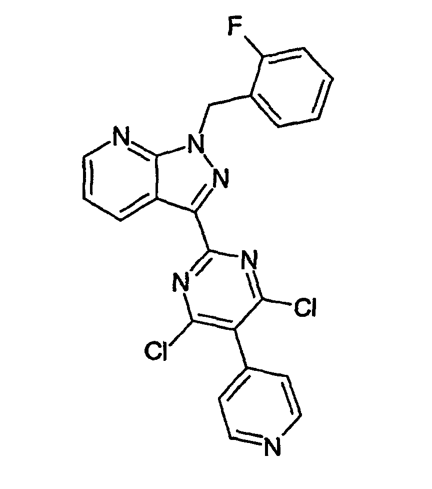

2.39 g (5.77 mmol) 2-[1-(2-Fluorobenzyl)-1H-pyrazolo[3,4-b]pyridin-3-yl]-5-(4-pyridinyl)-4,6-pyrimidindiol aus Bsp. IV werden in 10 ml Phosphorylchlorid gelöst. Dazu gibt man 3 Tropfen DMF und läßt 3h unter Rückfluß rühren. Zur Aufarbeitung engt man die Reaktionslösung ein und trocknet am Hochvakuum.
- Ausbeute.:: 0.67 g (24 %)
- LC-MS:: Rₜ = 4.34 min (Methode A).
MS (ESI pos.), *m*/*z* = 451 ([M+H]⁺, Cl₁).

### Beispiele

### 1. 2-[1-[(2-fluorophenyl)methyl]-1H-pyrazolo[3,4-b]pyridin-3-yl]-5-(4-pyridinyl)-4-pyrimidinamin

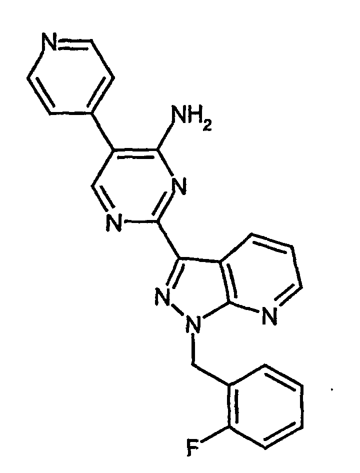

0.50 g (1.9 mmol) 1-(2-Fluorobenzyl)-1H-pyrazolo[3,4-b]pyridin-3-carboximidamid aus Bsp. III und [(Dimethylamino)methylen]-pyridineacetonitril (0.32 g, 1.9 mmol) aus Bsp. I wurden in Xylol suspendiert und mit BF₃*OEt₂ (71 µl, 79 mg, 0.56 mmol, 0.3 Äquiv.) versetzt. Nach 19 h bei 140°C ließ man auf Raumtemperatur abkühlen und engte im Vakuum ein. Die Titelverbindung konnte durch Flash-Chromatographie an Kieselgel (CH₂Cl₂:MeOH 20:1) und anschließendes Ausrübren in Acetonitril gereinigt werden.
- Ausbeute:: 0.24 g (33 %)
- R_{f}-Wert:: 0.17 (EE/MeOH 20:1)
- Fp:: 254°C
- Retentionzeit:: 2.7 min (Säule: Symmetry, C-18, 3.5 µm, 50X2.1 mm, Fluss 0.5 ml/min, 40°C, Gradient: Wasser (+0.1 % Ameisensäure):Acetonitril (+0.1 % Ameisensäure) bei 0 min: 90:10, bei 7.5 min 10:90))
- ¹H-NMR:: (300 MHz, D₆-DMSO), δ = 5.81 (s, 2H, CH₂), 7.0-7.6 (m, 9 H, Ar-H, NH₂), 8.64 (m_{c}, 3 H, Ar-H), 9.05 (d, 1 H, Ar-H)
- MS:: (ESI pos.), *m*/*z* = 398 ([M+H]⁺), (ESI neg.), *m*/*z* = 396 ([M-H]⁺)

### 2. 2-[1-[(2-Fluorophenyl)methyl]-1H-pyrazolo[3,4-b]pyridin-3-yl]-5-(4-pyridinyl)-4-pyrimidinamin

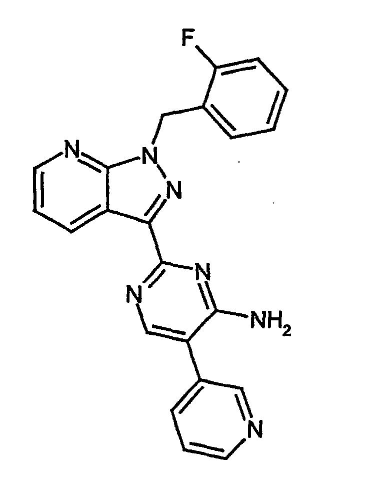

4.00 g (14.9 mmol) 1-(2-Fluorobenzyl)-1H-pyrazolo[3,4-b]pyridin-3-carboximidamid aus Bsp. III und 3-[(Dimethylamino)methylen]-pyridinacetonitril (2.57 g, 14.9 mmol) aus Bsp. II wurden in Xylol suspendiert. Nach 12 h bei 120°C ließ man auf Raumtemperatur abkühlen und filtrierte vom ausgefallenen Niederschlag. Die Mutterlauge wurde per präparativer HPLC (Säule: Cromsil 120 ODS, C-18, 10 µm, 250x30 mm, Fluss 50 ml/min, Raumtemperatur, Gradient: Wasser Acetonitril bei 0 min: 90: 10, bei 28 min 5:95) gereinigt. Der Reinigungsvorgang musste wiederholt werden.
- Ausbeute:: 0.024 g (0.4 %)
- R_{f}-Wert:: 0.17 (EE/MeOH 20:1)
- ¹H-NMR:: (200 MHz, D₆-DMSO), δ = 5.81 (s, 2H, OCH₂), 6.95-7.6 (m, 8 H, Ar-H, NH₂), 7.92 (dt, 1 H, Ar-H), 8.21 (S, 1H, Ar-H), 8.6-8.75 (m, 2 H, Ar-H), 9.03 (dd, 1 H, Ar-H).
- LCMS:: Ret.-zeit: 2.66 min (Säule: Symmetry, C-18, 3.5 µm, 50X2.1 mm, Fluss 0.5 ml/min, 40°C, Gradient: Wasser (+0.1 % Ameisensäure):Acetonitril (+0.1 % Ameisensäure) bei 0 min: 90:10, bei 7.5 min 10:90)); MS: (ESI pos.), *m*/*z* = 398 ([M+H]⁺), (ESI neg.), *m*/*z* = 396 ([M-H]⁺)

### 3. 6-Chloro-2-[1-(2-fluorobenzyl)-1H-pyrazolo[3,4-b]pyridin-3-yl]-5-(4-pyridinyl)-4-pyrimidinylamin

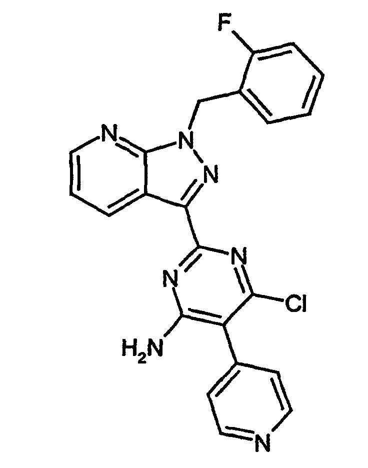

200 mg (0.443 mmol) 3-[4,6-Dichloro-5-(4-pyridinyl)-2-pyrimidinyl]-1-(2-fluorobenzyl)-1H-pyrazolo-[3,4-b]pyridin aus Bsp. V werden in 5 ml 25 %iger wäßriger Ammoniaklösung. suspendiert und im Autoklaven bei 140 °C und Eigendruck über Nacht gerührt. Die Mischung wurde dreimal mit Dichlormethan, extrahiert und die vereinigten Extrakte über Magnesiumsulfat getrocknet und zur Trockne eingeengt. Der Rückstand wurde über Kieselgel mit Dichlormethan/Methanol 30:1 chromatographiert. Zur weiteren Reinigung wurde das Rohprodukt über eine präparative HPLC gereinigt (Methode B).
- Ausbeute.:: 34 mg (15 %)
- R_{f}: 0.45 (CH₂Cl₂/MeOH 20:1)
- ¹H-NMR:: (300 MHz, D₆-DMSO), δ = 5.85 (s, 2H, CH₂), 7.10-7.48 (m, 9H, 7Ar-H und NH₂), 8.61 - 8.75 (m, 3H, Ar-H), 8.99 (dd, 1H, Ar-H).
- LC-MS:: Rₜ = 3.55 min (Methode A).
MS (ESI pos.), *m*/*z* = 432.3 ([M+H]⁺), 885.2 ([2M+Na]⁺).

### 4. 2-[1-(2-Fluorobenzyl)-1H-pyrazolo[3,4-b]pyridin-3-yl]-5-(4-pyridinyl)-4,6pyrimidindiamin

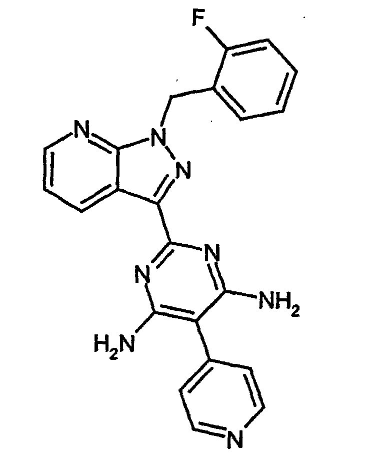

200 mg (0.443 mmol) 3-[4,6-Dichloro-5-(4-pyridinyl)-2-pyrimidinyl]-1-(2-fluoro-benzyl)-1H-pyrazolo-[3,4-b]pyridin aus Bsp. V werden in 5 ml 25%iger wäßriger Ammoniaklösung. suspendiert und im Autoklaven bei 140 °C und Eigendruck über Nacht gerührt. Die Mischung wurde dreimal mit Dichlormethan, extrahiert und die vereinigten Extrakte über Magnesiumsulfat getrocknet und zur Trockne eingeengt. Der Rückstand wurde über Kieselgel mit Dichlormethan/Methanol 30:1 chromatographiert. Zur weiteren Reinigung wurde das Rohprodukt über eine präparative HPLC gereinigt (Methode B).
- Ausbeute:: 45 mg (20 %)
- R_{f}: 0.30 (CH₂Cl₂/MeOH 20:1)
- ¹H-NMR:: (300 MHz, D₆-DMSO), δ = 5.82 (s, 2H, CH₂), 6.02 (br.s, 4H, NH₂), 7.08-7.48 (m, 7H, Ar-H), 8.57 - 8.68 (m, 3H, Ar-H), 9.13 (dd, 1H, Ar-H).
- LC-MS:: Rₜ = 2.55 min (Methode A).
MS (ESI pos.), *m*/*z* = 413.3 ([M+H]⁺), 847.8 ([2M+Na]⁺).

## Patentansprüche

1. Verbindungen der Formel (I) 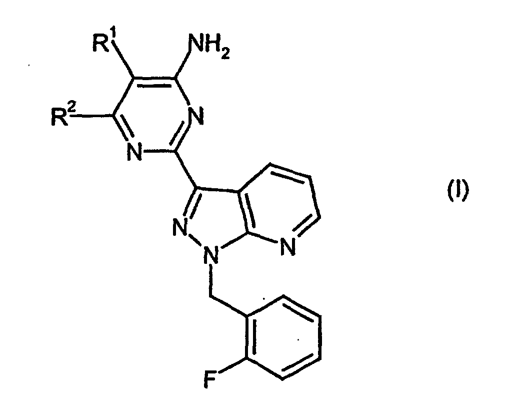 worin
R¹ für 4-Pyridinyl oder 3-Pyridinyl steht;
R² für H, NH₂ oder Halogen steht;
sowie Salze, und Hydrate davon.

2. Verbindungen nach Anspruch 1,
worin
R¹ für 4-Pyridinyl oder 3-Pyridinyl steht;
R² für H, NH₂ oder Cl steht;
sowie Salze, und Hydrate davon.

3. Verbindungen nach Anspruch 1,
worin
R¹ für 4-Pyridinyl oder 3-Pyridinyl steht;
R² für H steht;
sowie Salze, und Hydrate davon.

4. Verfahren zur Herstellung der Verbindung der Formel 1, umfassend die Umsetzung der Verbindung der Formel (II) 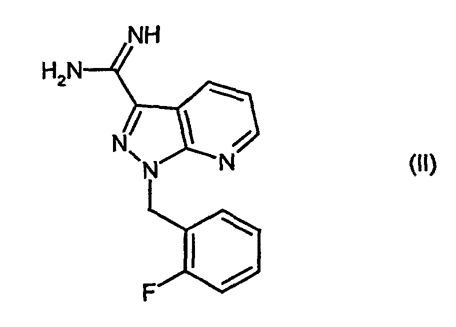
A) mit einer Verbindung der Formel (III) 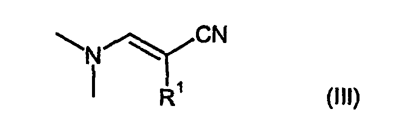 wobei
R¹ wie vorstehend definiert ist;
gegebenenfalls in einem organischen Lösungsmittel unter Erhitzen zur Verbindung der Formel (I);
oder
B) mit einer Verbindung der Formel (IV) 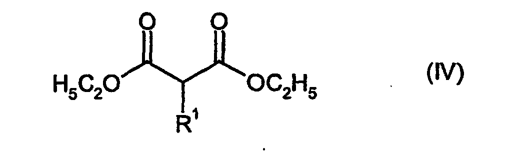
wobei
R¹ wie vorstehend definiert ist;
in einem organischen Lösungsmittel unter Erhitzen zu Verbindungen der Formel (V) 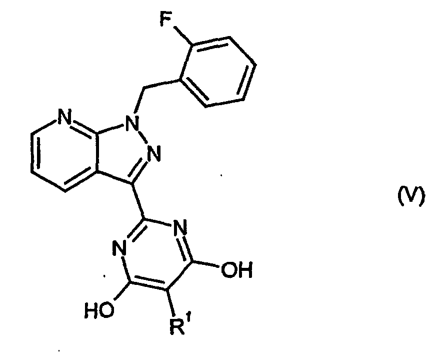 wobei
R¹ wie vorstehend definiert ist;
anschließend mit einem Halogenierungsmittel zu Verbindungen der Formel (VI) 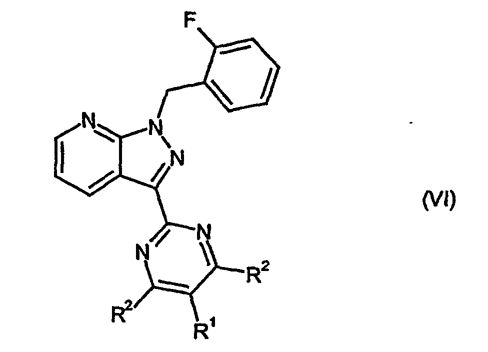 wobei
R¹ wie vorstehend definiert ist;
R² für Halogen steht;
sowie abschließend mit wäßriger Ammoniaklösung unter Erhitzen und erhöhtem Druck.

5. Verbindung der Formel (I) zur Behandlung von Krankheiten.

6. Arzneimittel enthaltend mindestens die Verbindung der Formel (I) gemäß Anspruch 1.

7. Verfahren zur Herstellung von Arzneimitteln, **dadurch gekennzeichnet, dass** man die Verbindung der Formel (I) gemäß Anspruch 1, gegebenenfalls mit üblichen Hilfs- und Zusatzstoffen in eine geeignete Applikationsform überführt.

8. Arzneimittel enthaltend die Verbindung der Formel (I) gemäß Anspruch 1 in Kombination mit organischen Nitraten oder NO-Donatoren.

9. Arzneimittel enthaltend die Verbindung der Formel (I) gemäß Anspruch 1 in Kombination mit Verbindungen, die den Abbau von cyclischen Guanosinmonophosphat (cGMP) inhibieren.

10. Verwendung der Verbindung der Formel (I) gemäß Anspruch 1 bei der Herstellung von Arzneimitteln zur Behandlung von Herz-Kreislauf-Erkrankungen.

11. Verwendung der Verbindung der Formel (I) gemäß Anspruch 1 bei der Herstellung von Arzneimitteln zur Behandlung von Hypertonie.

12. Verwendung der Verbindung der Formel (I) gemäß Anspruch 1 bei der Herstellung von Arzneimitteln zur Behandlung von thromboembolischen Erkrankungen und Ischämien.

13. Verwendung der Verbindung der Formel (I) gemäß Anspruch 1 bei der Herstellung von Arzneimitteln zur Behandlung von sexueller Dysfunktion.

14. Verwendung der Verbindung der Formel (I) gemäß Anspruch 1 bei der Herstellung von Arzneimitteln mit antiinflammatorischen Eigenschaften.

15. Verwendung von Verbindungen der allgemeinen Formel (I) gemäß Anspruch 1 bei der Herstellung von Arzneimitteln zur Behandlung von Erkrankungen des Zentralnervensystems.

16. Verwendung gemäß einem der Ansprüche 8 bis 13, wobei die Verbindung der Formel (I) gemäß Anspruch 1 in Kombination mit organischen Nitraten oder NO-Donatoren oder in Kombination mit Verbindungen, die den Abbau von cyclischen Guanosinmonophosphat (cGMP) inhibieren, eingesetzt wird.

## Claims

1. Compounds of the formula (I) 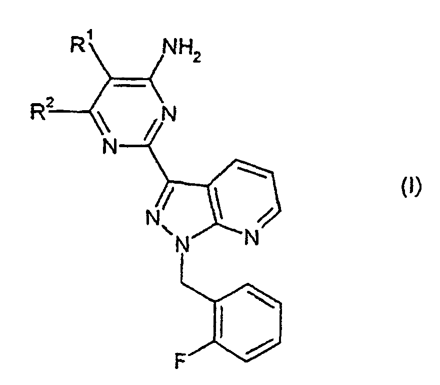 in which
R¹ represents 4-pyridinyl or 3-pyridinyl;
R² represents H, NH₂ or halogen;
and salts and hydrates thereof.

2. Compounds according to Claim 1,
in which
R¹ represents 4-pyridinyl or 3-pyridinyl;
R² represents H, NH₂ or Cl;
and salts and hydrates thereof.

3. Compounds according to Claim 1,
in which
R¹ represents 4-pyridinyl or 3-pyridinyl;
R² represents H;
and salts and hydrates thereof.

4. Process for the preparation of the compound of the formula I, comprising the reaction of the compound of the formula (II) 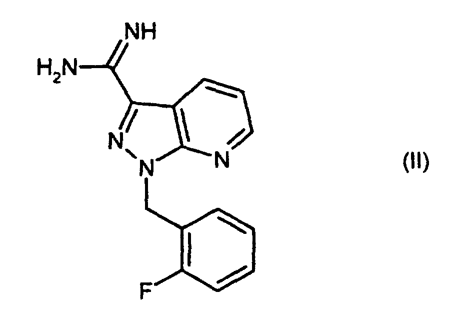
A) with a compound of the formula (III) 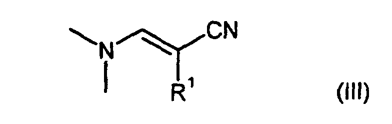 where
R¹ is as defined above;
if appropriate in an organic solvent, with heating to give the compound of the formula (I);
or
B) with a compound of the formula (IV) 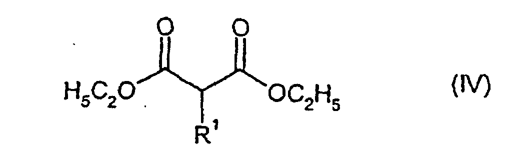
where
R¹ is as defined above;
in an organic solvent, with heating to give compounds of the formula (V) 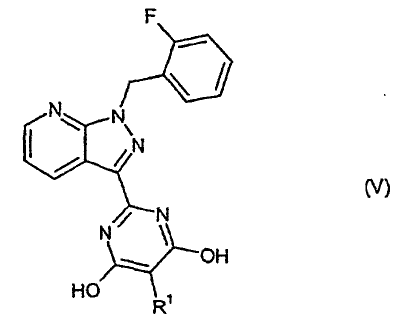 where
R¹ is as defined above;
subsequently with a halogenating agent to give compounds of the formula (VI) 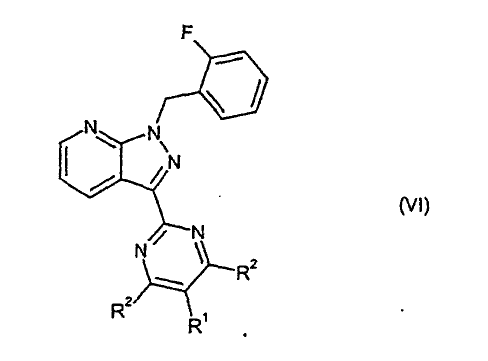 where
R¹ is as defined above;
R² represents halogen;
and also finally with aqueous ammonia solution with heating under elevated pressure.

5. Compound of the formula (I) for the treatment of diseases.

6. Medicaments comprising at least one compound of the formula (I) according to Claim 1.

7. Process for the production of medicaments, **characterized in that** the compound of the formula (I) according to Claim 1 is brought into a suitable administration form, if appropriate using customary excipients and additives.

8. Medicaments comprising the compound of the formula (I) according to Claim 1 in combination with organic nitrates or NO donors.

9. Medicaments comprising the compound of the formula (I) according to Claim 1 in combination with compounds which inhibit the breakdown of cyclic guanosine monophosphate (cGMP).

10. Use of the compound of the formula (I) according to Claim 1 in the production of medicaments for the treatment of cardiovascular disorders.

11. Use of the compound of the formula (I) according to Claim 1 in the production of medicaments for the treatment of hypertension.

12. Use of the compound of the formula (I) according to Claim 1 in the production of medicaments for the treatment of thromboembolic disorders and ischaemias.

13. Use of the compound of the formula (I) according to Claim 1 in the production of medicaments for the treatment of sexual dysfunction.

14. Use of the compound of the formula (I) according to Claim 1 in the production of medicaments having anti-inflammatory properties.

15. Use of compounds of the general formula (I) according to Claim 1 in the production of medicaments for the treatment of disorders of the central nervous system.

16. Use according to one of Claims 8 to 13, where the compound of the formula (I) according to Claim 1 is employed in combination with organic nitrates or NO donors or in combination with compounds which inhibit the breakdown of cyclic guanosine monophosphate (cGMP).

## Revendications

1. Composés de formule (I) 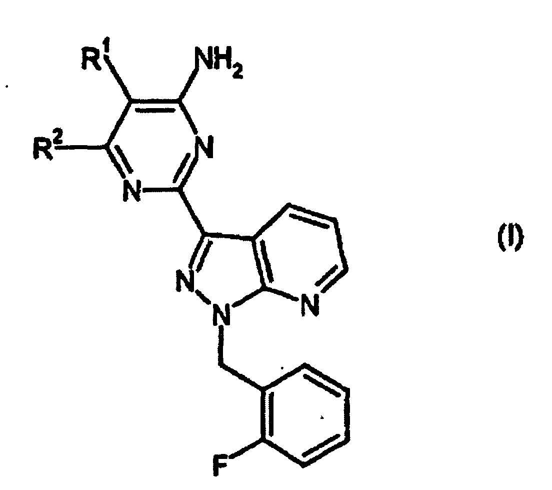 dans laquelle
R¹ désigne un radical 4-pyridinyle ou 3-pyridinyle;
R² désigne NH, NH₂ ou un halogène,
ainsi que des sels et des hydrates de ceux-ci.

2. Composés selon la revendication 1,
dans lesquels
R¹ désigne un radical 4-pyridinyle ou 3-pyridinyle;
R² désigne H, NH₂ ou Cl,
ainsi que des sels et des hydrates de ceux-ci.

3. Composés selon la revendication 1,
dans lesquels
R¹ désigne un radical 4-pyridinyle ou 3-pyridinyle;
R² désigne H,
ainsi que des sels et des hydrates de ceux-ci.

4. Procédé de préparation de composés de formule 1, comprenant la mise en réaction du composé de formule (II) 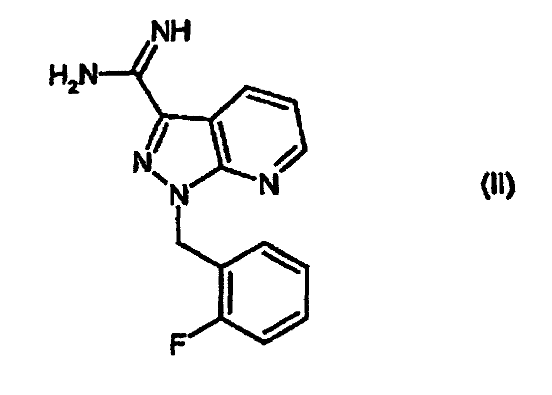
A) avec un composé de formule (III) 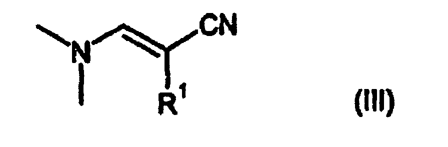 dans laquelle
R¹ répond à la définition précédente;
éventuellement dans un solvant organique avec chauffage pour former le composé de formule (I);
ou
B) avec un composé de formule (IV) 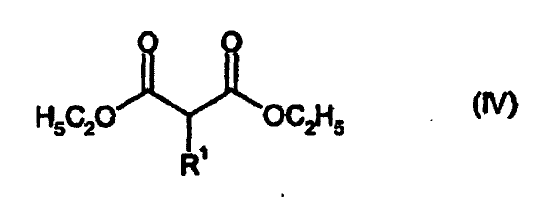
dans laquelle
R¹ répond à la définition précédente;
dans un solvant organique avec chauffage pour former des composés de formule (V); 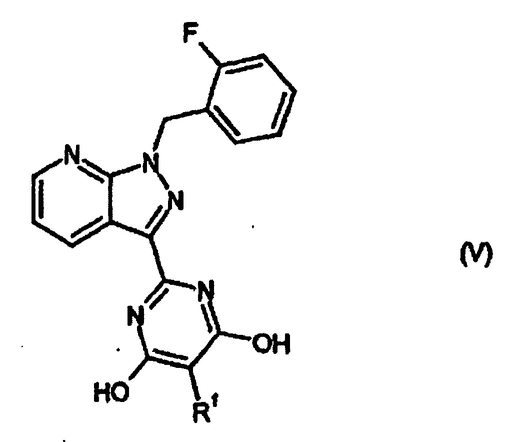 dans laquelle
R¹ répond à la définition précédente;
puis, avec un agent d'halogénation en composés de formule (VI) 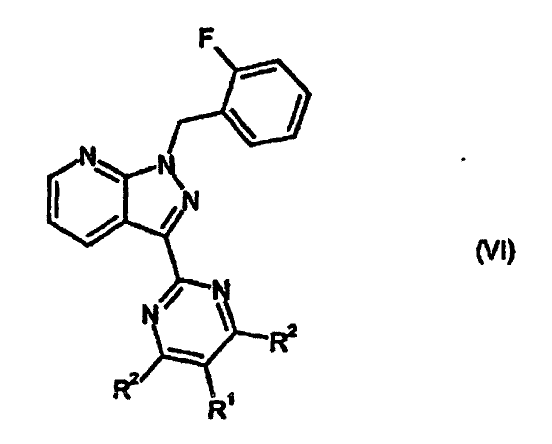 dans laquelle
R¹ répond à la définition précédente;
R² désigne un halogène;
ainsi que, pour terminer, avec une solution aqueuse d'ammoniaque sous chauffage et pression élevée.

5. Composé de formule (I) pour le traitement de maladies.

6. Médicament contenant au moins le composé de formule (I) selon la revendication 1.

7. Procédé de préparation de médicaments, **caractérisé en ce que** l'on transforme le composé de formule (I) selon la revendication 1, éventuellement avec des additifs et adjuvants courants, en une forme d'administration adéquate.

8. Médicament contenant le composé de formule (I) selon la revendication 1 en combinaison avec des donneurs de NO ou des nitrates organiques.

9. Médicament contenant le composé de formule (I) selon la revendication 1 en combinaison avec des composés qui inhibent la décomposition de monophosphate de guanosine (cGMP) cyclique.

10. Mise en oeuvre du composé de formule (I) selon la revendication 1 en cas de fabrication de médicaments pour le traitement de maladies cardio-vasculaires.

11. Mise en oeuvre du composé de formule (I) selon la revendication 1 pour la préparation de médicaments en vue du traitement de l'hypertonie.

12. Mise en oeuvre du composé de formule (I) selon la revendication 1 pour la préparation de médicaments en vue du traitement de maladie thrombo-emboliques et d'ischémies.

13. Mise en oeuvre du composé de formule (I) selon la revendication 1 pour la préparation de médicaments en vue du traitement de dysfonctionnements sexuels.

14. Mise en oeuvre du composé de formule (I) selon la revendication 1 pour la préparation de médicaments aux propriétés anti-inflammatoires.

15. Mise en oeuvre de composés de formule générale (I) selon la revendication 1 pour la préparation de médicaments en vue du traitement de maladies du système nerveux central.

16. Mise en oeuvre selon l'une des revendications 8 à 13, dans laquelle le composé de formule (I) selon la revendication (1 ) est utilisé en combinaison avec des donneurs de NO ou des nitrates organiques ou en combinaison avec des composés qui inhibent la décomposition de monophosphate de guanosine (cGMP) cyclique.
